(19) Europäisches Patentamt
European Patent Office
Office européen des brevets

(11) Veröffentlichungsnummer: **0 199 840**
**B1**

(12) # EUROPÄISCHE PATENTSCHRIFT

(45) Veröffentlichungstag der Patentschrift: **18.04.90**

(51) Int. Cl.[5]: **A 61 K 31/13**

(21) Anmeldenummer: **85105395.9**

(22) Anmeldetag: **03.05.85**

(54) **Verwendung eines Diamins zur Herstellung eines Erythrozyten-Weichmachers.**

(43) Veröffentlichungstag der Anmeldung:
**05.11.86 Patentblatt 86/45**

(45) Bekanntmachung des Hinweises auf die Patenterteilung: **18.04.90 Patentblatt 90/16**

(84) Benannte Vertragsstaaten:
**AT BE CH DE FR GB IT LI LU NL SE**

(56) Entgegenhaltungen:
**US-A-4 383 997**

**CHEMICAL ABSTRACTS, Band 87, Nr. 17, 24. Oktober 1977, Seite 76, Nr. 127412n, Columbus, Ohio, US; N.H. HAMMOSHI: "Effect of piperazine on the intact mammalian cardiovascular system", ANN. COLL. MED. MOSUL 1977, 8(1), 29-37**
**CHEMICAL ABSTRACT, Band 102, 10. Juni 1985, Seite 20, Nr. 197539m, Columbus, Ohio, US; K. AISAKA et al.: "The effects of piperidine and its related substances on blood vessels", & JPN. J. PHARMACOL. 1985, 37(4), 345-53**
**CHEMICAL ABSTRACTS, Band 52, Nr. 21, 10. November 1958, Spalten 18899f-18900c, Columbus, Ohio, US; L. BUCHEL et al.: "Cardiovascular and synaptotropic properties of some branched amines", & THERAPIE 11, 821-47(1956)**

(73) Patentinhaber: **Barnikol, Wolfgang, Prof. Dr.Dr.**
**Lanzelhohl 66**
**D-6500 Mainz-Bretzenheim (DE)**

(72) Erfinder: **Barnikol, Wolfgang, Prof. Dr.Dr.**
**Lanzelhohl 66**
**D-6500 Mainz-Bretzenheim (DE)**

(74) Vertreter: **Ratzel, Gerhard, Dr.**
**Seckenheimer Strasse 36a**
**D-6800 Mannheim 1 (DE)**

**EP 0 199 840 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Bekanntlich sind Durchblutungsstörungen, insbesondere jedoch die Verminderung der Durchblutung eine entscheidende Basis für eine Reihe von Krankheitssyndromen; dies gilt beispielsweise für den Herzinfarkt, die Hirnerweichung, die Claudicatio intermittens, bei Morbus Raynaud, Morbus Winiwater-Bürger, bei enteritischen Ischämien, ferner bie diabetischen Mikroangiopathien, zum Beispiel des Auges und der Niere sowie bei Arteriosklerose. Diese Aufzählungen sind beispielhafter Natur.

Man hat zwischen Einengung der größeren Gefäße (Arterien) und einer mikrozirkulatorischen Störung zu unterscheiden. Bei der letzteren kommt es zu einer dratischen Verminderung der kapillären Perfusion, wobei die globale Durchblutung weitgehend aufrecht erhalten sein kann; die Perfusion erfolgt in diesem Fall hautpsächlich über funktionelle Anastomosen. Für die kapilläre Perfusion ist die Flexibilität der Erythrozyten von großer Bedeutung; der mittlere innere Durchmesser der Kapillaren beträgt nämlich 5 µm, während ruhende Erythrozyten 8 µm breit sind. Damit ein Erythrozyt eine Kapillar passieren kann, muß er sich erheblich verformen können. Dementsprechend besitzen normale Erythrozyten eine große Flexibilität. Bei einigen Erkrankungen wurde eine verringerte Verformbarkeit der Erythrozyten gemessen. Dies gilt beispielsweise für eine Reihe von Fällen des Diabetes mellitus, der im Endstadium durch Versorungsstörungen bis hin zur Gangrän gekennzeichnet ist. Als Literatur ist hier beispielsweise zu nennen: Juhan et al., The Lancet (1982) 535—537; Barnikol et al., Funkt. Biol. Med. *3* (1984) 249—252.

Analoges gilt auch für Hypertoniker, hier ist folgende Literaturstelle zu nennen: Barnikol et al., Funkt Biol. Med. *4* (1985) 61—66.

Eine ausreichende kapilläre Perfusion ist deshalb so bedeutsam, weil sie allein die Voraussetzung für eine regelrechte Ver- und Entsorgung der Zellen ist und nicht etwa die Anastomosen-Perfusion, obwohl eine ausreichende arterielle Durchblutung für die kapilläre Perfusion permissiv ist.

Dabei handelt es sich nicht nur um den Sauerstoff, deren Träger die Erythrozyten sind, sondern, wenn die kapilläre Perfusion durch eine Versteifung der Kapillaren vermindert ist, sogar um das Substratangebot insgesamt, also beispielsweise auch um Glukose. Insoweit kommt den Erythrozyten daher eine Schlüsselstellung in Bezug auf die gewebliche Versorgung zu.

Aus dem oben Dargelegten ergeben sich zwei Stragegien für die Erhöhung der Perfusion:

1. Eine Erweiterung der Gefäße durch Relaxierung der glatten Gefäßmuskulatur (vasaler Angriffspunkt).

2. Eine Erhöhung der Erythrozyten-Verformbarkeit durch Weichmacher (intravasaler Angriffspunkt).

Mit ersterem wird vorwiegend an Gefäßen, die reichlich mit Muskulatur ausgestattet sind, nämlich den Arterien einschließlich der Arteriolen, eine Wirkung erzielt, mit letzterem dagegen vorwiegend an Gefäßen mit einem kleineren inneren Durchmesser als 8 µm, bei welchem die Perfusion entscheidend durch die erythrozytäre Flexibilität bestimmt wird und welche, wenn üherhaupt, dann eine stark rarifizierte glatte Muskulatur besitzen.

Bisher wurde von einer Substanz, nämlich dem 3,7-Dimethyl-1-(5-oxohexyl)-xanthin (Pentoxifyllin), berichtet, daß sie die Fließeigenschaften der Erythrozyten in vivo verbessert. Pentoxifyllin ist die Wirksubstanz des zur Zeit unter dem Namen Trental® von der Firma Chemische Werke Albert, Wiesbaden-Biebrich, in den Handel gebrachten Pharmazeutikums.

Es wurde nun gefunden, daß es, um die Erythrozyten-weichmachende Wirkung einer Substanz mit Hilfe eines Filtrationstests festzustellen, unerlässlich ist, die Erythrozyten quantitativ von den andern Blutzellen abzutrennen, weil auch sehr geringe Zellverunreinigungen den Test übermäßig stark stören Daher wurde ein Testverfahren einschließlich eines Reinigungsverfahrens mit Hilfe der Dichtezentrifugation in synthetischem Polysaccharid (Ficoll® 400, Deutsche Pharmazia, Freiburg, Bundesrepublik Deutschland) erarbeitet; als Literaturstelle sei hingewiesen auf: Barnikol et al., Funkt, Biol. Med. *1* (1982), 242—244.

Im Prinzip wird hierbei die sogenannte spezifische Filtrierzeit (FZsp) einer 8%igen Erythrozytensuspension an 5 µm Polycarbonat (Nuclepore-®) Membran-filtern bestimmt. Das Meßverfahren wurde insofern gegenüber der ursprünglichen Vorschrift modifiziert, als nunmehr lediglich die Anfangsgeschwindigkeit gemessen wird, also je nach Dauer variable Tropfenzahlen vermessen werden, sowie der Zeitbedarf für eine bestimmte Tropfenzahl, nämlich 10, bereichnet wurde.

Es gilt die Formel

$$FZsp=(t-t_o)/t_o$$

wobei:

t=Filtrierzeit für 10 Tropfen der Suspension

$t_o$=Filtrierzeit für 10 Tropfen des Suspensionsmittels

FZsp ist ein Maß für die Steifigkeit.

Mit diesem in vitro-Testverfahren erwies sich Pentoxifyllin von angegebenen Wirkspiegeln (0,1 mmol/l) bit 15 mmol/l bei 22°C und pH=7,40 als unwirksam: FZsp wurde nicht signifikant verkleinert.

Zum Stand der Technik sind die folgenden Druckschriften zu nennen. Die US—A—4 383 997 betrifft Verbindungen, die den Zustrom extrazellulären Ca++ in rote Blutkörperchen inhibieren und den intrazellulären Gehalt an ATP erhöhen.

Dabei handelt es sich um an beiden Stickstoffatomen substituierte Piperazine, wobei die Substituenten einerseits Cinnamyl-Reste und andererseits Benzhydryl-Reste darstellen.

Diese N-substituierten Piperazin-Derivate dienen als Behandlungsmittel für Pferde; ein Hinweis auf die reduzierte Deformierbarkeit von Erythrozyten-Zellmembranen wird gegeben.

Die Literaturstelle "Chemical Abstracts", Band

87, Nr. 17, 24.Oktober 1977, Seite 76, Nr 127412n, betrifft die Wirkung von unsubstituiertem Piperazin auf das Kreislaufsystem von Ratten. Dabei wird in zweiphasiger Weise zunächst eine Reduzierung des Blutdrucks und der Herzschlagrate bewirkt, gefolgt von einem vorübergehenden Anstieg dieser beiden Werte. Hier handelt es sich demnach um einen kardiovaskulären Angriffspunkt, keinesfalls um einen intravasalen in oben genanntem Sinne.

Die Literaturstelle "Chemical Abstracts", Band 52, Nr 21, 10. November 1958, Spalten 18899f—18900c, betrifft die kardiovaskuläre Wirkung von N-substituierten Piperidinen, welche einen vorübergehenden Blutdruckanstieg ohne Gefäßverengung bewirken. Somit handelt es sich hier um kardiale Wirkungen, also weder um einen vasalen noch intravasalen Angriffspunkt.

Die Verbindungen des Standes der Technik bewirken eine ungenügende Deformierbarkeit der Erythryzyten; somit ist mit ihnen eine unzureichende Wirkung der erhöhten Sauerstoffversorgung in Bereichen kapillarer Perfusion möglich.

Der Erfindung liegt nun die Aufgabe zugrunde, geeignete Substanzen zu liefern, die in der Lage sind, eine Erhöhung der kapillaren Perfusion zu bewirken, d.h. die das Vermögen besitzten, eine normale oder eine pathologisch verringerte Flexibilität der Erythrozyten spruhghaft zu steigern.

Diese vorliegender Erfindung zugrunde liegende Aufgabe wird dadurch gelöst, daß die Verwendung wenigstens eines Diamins aus der Gruppe Dimethylaminoethylamin, Tetramethylethylendiamin, Tetraethylethylendiamin zur Herstellung eines Ezthrozyten-Weichmachers erfolgt.

Beansprucht wird weiterhin die Verwendung wenigstens eins erfindungsgemäßen Diamins in Form von Infusionslösungen, Kapseln, Tabletten oder biologischem Puffer.

Diese Diamine werden den Erythrozytensuspensionen zur Erreichung des gewünschten Effekts, vorzugsweise des therapeutischen Effekts zugesetzt.

Dabei haben sich gemäß vorliegender Erfindung die drei Diamine

1. Dimethylaminoethylamin (DMAEA)
2. Tetramethylethylendiamin (TMEDA) und
3. Tetraethylethylendiamin (TEEDA)

als besonders wirksam erwiesen.

Alle diese Substanzen besitzen zwei Aminogruppen, die über eine oder zwei Methylenbrükken verknüpft sind; alle drei Substanzen liegen bei einem pH-Wert von 7,4 im halbtitrierten Zustand vor.

Beim TMEDA der Formel

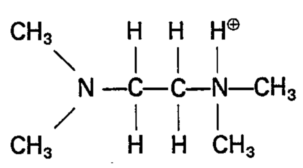

ist die Wirkung eventuell auf das gleichzeitige Vorhandensein einer quartären und einer freien Aminogruppe in einem bestimmten molekularen Abstand, zurückzuführen.

Die neuen erfindungsgemäßen Erythrozyten-Weichmacher weisen den überraschenden und fortschrittlichen Effekt der Verminderung der Erythrozytenrigidität und der Steigerung der Durchblutung verschiedener Organe bei zahlreichen Erkrankungen auf.

Ein weiterer Vorteil der erfindungsgemäßen Erythrozyten-Weichmacher, also der Diamine besteht darin, daß sie zugleich als biologische Puffer dienen können.

Das Wesen vorliegender Erfindung wird weiterhin anhand von Ausführungsbeispielen erläutert:

Ausführungsbeispiel 1: DMAEA

Erthrozyten von mehreren gesunden Probanden werden wie oben angegeben (Barnikol et al., 1982) präpariert und eine 8 Vol.-%ige Zellsuspension hergestellt unter Zugabe von Glukose (100 mg/dl). Den Suspensionen werden steigende Mengen von DMAEA von 0 bis 100 mmol/l zugesetzt. Die Proben werden 1,5 Stunden unter langsamen Schwenken belassen bei pH=7,4 und 22°C und danach FZsp gemessen: Bei 50 mmol/l wird FZsp auf 30% des Ausgangswertes verringert.

Ausführungsbeispiel 2: TMEDA

Ausführung wie in Ausführungsbeispiel 1, gleiche Wirkung.

Ausführungsbeispiel 3: TEEDA

Ausführung wie in Ausführungsbeispiel 1, gleich Wirkung.

**Patentansprüche**

1. Verwendung wenigstens eines Diamins aus der Gruppe Dimethylaminoethylamin, Tetramethylethylendiamin, Tetraethylethylendiamin zur Herstellung eines Erythrozyten-Weichmachers.

2. Verwendung wenigstens eines Diamins nach Anspruch 1 in Form von Infusionslösungen, Kapseln, Tabletten oder biologischem Puffer.

**Revendications**

1. Utilisation au moins d'une diamine de la groupe comportant diméthylaminoéthylamine, tetraméthyléthylène diamine, tetraéthyl-éthylène diamine pour la préparation d'un plastifiant (adourcisseur) pour érythrocytes.

2. Utilisation au moins d'une diamine selon la revendication 1 en forme de solutions pour infusions, capsules, comprimés ou tampon biologique.

**Claims**

1. Utilization of at least one diamine from the group comprising dimethyl-amino-ethylamine, tetramethylethylene-diamine, tetraethyl-ethylenediamine for the preparation of an erythrocyte-softener.

2. Utilization of at least one diamine according or biological buffer.